# FASCICULE DE BREVET EUROPEEN

(11) **EP 0 764 629 B1**
(45) Date de publication et mention de la délivrance du brevet: **08.12.1999**
(21) Numéro de dépôt: 96401981.4
(22) Date de dépôt: 18.09.1996
(51) Int. Cl.: C07C 69/54, C07C 67/14, C08F 20/30, C08F 220/30

(54) **Matériau composite à renfort fibreux et matrice obtenue par polymérisation de monomères acryliques, et sa fabrication**
Durch Fasern verstärkter Verbundwerkstoff und durch Polymerisation von Acrylmonomeren erthaltene Matrize und seine Herstellung
Fiber reinforced composite material and by polymerisation of acrylic monomers obtained matrix and its production

(30) Priorité: 20.09.1995 FR 9511038
(43) Date de publication de la demande: 26.03.1997
(73) Titulaire: AEROSPATIALE Société Nationale Industrielle, 75781 Paris Cédex 16 (FR)
(72) Inventeur: Dazens, Véronique, 33260 Pyla sur Mer (FR); Beziers, Daniel, 33160 Saint Medard en Jalles (FR); Chataignier, Evelyne, 33160 Saint Medard en Jalles (FR); Filliatre, Claude, 33400 Talence (FR); Villenave, Jean-Jacques, 33170 Gradignan (FR); Servens, Christian, 33160 St. Aubin de Medoc (FR)
(74) Mandataire: Des Termes, Monique

(56) Documents cités:
- US-A- 3 586 527
- US-A- 3 721 644
- PATENT ABSTRACTS OF JAPAN vol. 9, no. 119 (C-282), 23 Mai 1985 & JP 60 011409 A (MITSUBISHI RAYON KK), 21 Janvier 1985,

## Description

La présente invention a pour objet l'utilisation de monomères acryliques pour former la matrice de matériaux composites à renfort fibreux.

Elle s'applique notamment à la réalisation de matériaux composites destinés à l'aviation, qui doivent posséder des propriétés de vieillissement en atmosphère humide correspondant aux normes avioniques.

Des matériaux composites de ce type comprennent habituellement un renfort fibreux, constitué par exemple de fibres de carbone, de fibres de verre, ou de fibres d'aramide, par exemple Kevlar®, enrobé par une matrice de résine organique. Pour la préparation du matériau, on imprègne généralement le renfort fibreux par la résine à l'état liquide, puis on procède au durcissement de la résine. Les résines durcissables par rayonnement X ou électrons, c'est-à-dire polymérisables par ionisation, sont particulièrement intéressantes pour réaliser de tels matériaux, car elles durcissent rapidement, pratiquement sans élévation de température ; on évite ainsi des conditions de traitement qui pourraient être néfastes pour la qualité du composite.

Parmi les résines utilisables pour former la matrice de tels matériaux, les résines acryliques sont avantageuses car elles sont polymérisables par ionisation et présentent de bonnes propriétés thermiques.

Ainsi, on connait par les documents FR-A-2 581 991, FR-A-2 594 825 et EP-A-0 218 722, des compositions polymérisables à base de monomères tri- ou tétraacryliques ou méthacryliques, qui permettent l'obtention de polymères tridimensionnels, fortement réticulés, avec de bonnes performances thermiques. Ces monomères sont obtenus par réaction d'une diamine aromatique ou cycloaliphatique avec l'acrylate ou le méthacrylate de glycidyle. Aussi, de telles compositions ne conviennent pas pour la préparation de matériaux composites devant résister au vieillissement en atmosphère humide car leur structure contient des fonctions hydrophiles (groupes amine et hydroxyle) qui sont susceptibles de faire chuter les propriétés thermiques après vieillissement en atmosphère humide. De plus, la présence de telles fonctions entraîne une augmentation de viscosité des monomères, ce qui constitue un inconvénient pour leur introduction dans un renfort fibreux. Par ailleurs, il est nécessaire dans certains cas, d'effectuer un post traitement thermique en raison d'une polymérisation incomplète de la matrice, ce qui ne permet pas de profiter pleinement des avantages (rapidité et facilité de mise en oeuvre) de la polymérisation par ionisation.

La présente invention a précisément pour objet un matériau composite comprenant un renfort fibreux et une matrice obtenue par polymérisation de monomères acryliques ou méthacryliques durcissables sous rayonnement X ou électrons, qui pallient ces inconvénients et permettent d'obtenir un matériau composite présentant la résistance voulue au vieillissement en atmosphère humide (peu d'absorption d'eau avec maintien d'une température de transition vitreuse (Tg) élevée, de l'ordre de 120 à 170°C).

Selon l'invention, le matériau composite comprend un renfort fibreux et une matrice obtenue par polymérisation d'une composition durcissable par ionisation au moyen de rayons X ou d'électrons, constituée par un mélange d'un tétraacrylate de formule : 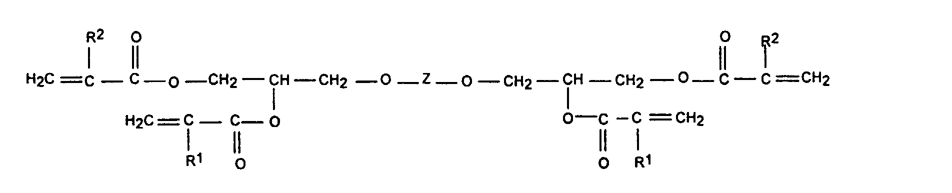 dans laquelle R¹ et R² qui peuvent être identiques ou différents, représentent H ou CH₃, et Z est un groupement bivalent comportant un ou deux cycles aromatiques ou aliphatiques,
avec
1) un diacrylate et/ou triacrylate de formule : 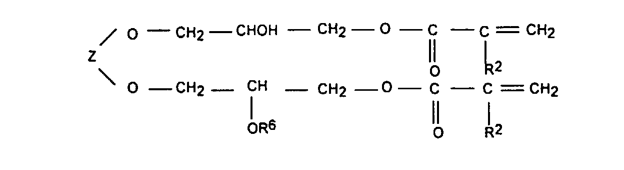 dans laquelle Z et R² ont les significations données ci-dessus et R⁶ représente H ou le groupe de formule : 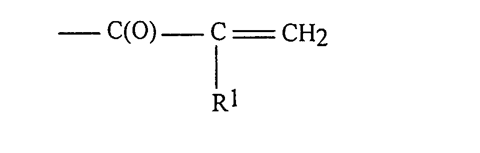 dans laquelle R¹ représente H ou CH₃, ou
2) un diluant acrylique monofonctionnel.

On précise que dans le présent texte le terme acrylate s'applique également au méthacrylate.

Dans le matériau composite de l'invention, le renfort fibreux, peut être formé de fibres de carbone, de fibres de verre, ou de fibres d'aramide tel que le Kevlar®.

Ce renfort fibreux peut être sous différentes formes, par exemple sous la forme de fibres empilées, sous la forme de nappes bidirectionnelles ou sous la forme de tissus tri- ou pluridirectionnels.

Généralement, ce renfort fibreux représente 55 à 75 % en volume du matériau composite, le reste (25 à 45 %) étant occupé par la matrice de résine acrylique durcie.

On peut en particulier réaliser un matériau composite comprenant 70 % en volume de renfort fibreux et 30 % en volume de résine.

Les tétraacrylates utilisés dans l'invention sont particulièrement intéressants car ils comportent quatre fonctions réactives acryliques qui permettent d'obtenir une densité de réticulation du réseau polymère élevée et donc d'augmenter la température de transition vitreuse (Tg) du matériau. De plus, ils ne comportent pas de fonction hydrophile (hydroxyle ou amine) susceptible d'affaiblir le comportement du matériau en atmosphère humide.

Dans ces tétraacrylates, la partie centrale constituée par le groupement Z peut être de différents types, par exemple formée d'un enchaînement :

-R³-R⁴-R³-

dans lequel R⁴ est un groupe bivalent, par exemple 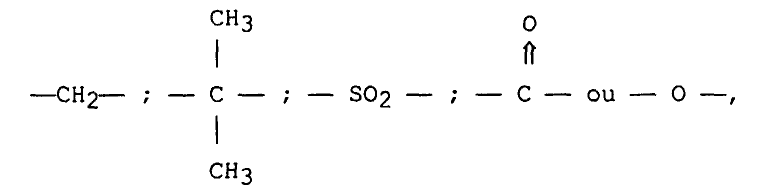 et les R³ sont des cycles aromatiques.
Le groupement Z peut aussi être constitué par un seul cycle aromatique ou aliphatique tel que :

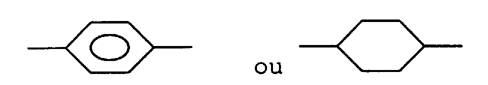

Selon un mode de réalisation préféré de l'invention, le groupement Z du tétraacrylate est dérivé du bis-phénol A ou du bis-phénol F. Dans ce cas, Z répond à la formule : 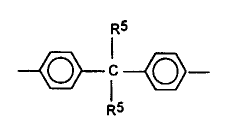 dans laquelle R⁵ représente un atome d'hydrogène ou un groupe méthyle.

Ces monomères tétraacryliques, peuvent être obtenus à partir de diacrylates de formule : 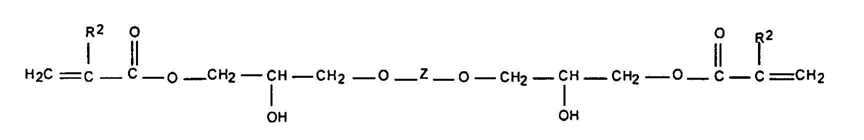 dénommés BIS-GA ou BIS-GMA (méthacrylate)
par réaction de ceux-ci avec un chlorure d'acide de formule : 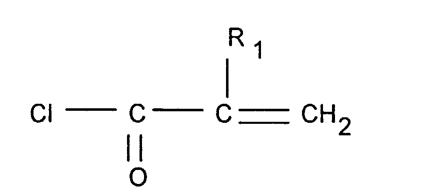 dans lesquelles R¹, R² et Z ont les significations données ci-dessus.

Cette réaction peut être effectuée en une seule étape, en milieu basique, par exemple en présence d'une amine tertiaire telle que la triéthylamine. Généralement, on opère dans un solvant organique tel que le chloroforme. Au cours de cette réaction, plusieurs produits peuvent être formés ; ceux-ci sont le monomère tétraacrylique et le monomère triacrylique.

En faisant varier les conditions expérimentales, les quantités relatives de diacrylate et de chlorure d'acide, on peut maitriser la synthèse et l'orienter soit vers la production du produit tétraacrylique pur sans purification ultérieure, soit sur l'obtention d'un mélange de produits triacrylique et tétraacrylique. Cette dernière possibilité est intéressante car comme on le verra plus loin, elle permet d'obtenir directement des compositions durcissables convenant à la réalisation de la matrice d'un matériau composite.

Les diacrylates de départ utilisés pour la synthèse du tétraacrylate peuvent être obtenus à partir de l'éther de glycidyle du diol correspondant HO-Z-OH, par réaction avec l'acide acrylique ou méthacrylique. Ainsi, dans le cas où Z représente des dérivés du bis-phénol A, ce diacrylate ou époxyacrylate qui est un produit commercial, est formé à partir de l'éther de glycidyle du bis-phénol A et de l'acide acrylique ou méthacrylique.

Les tétraacrylates utilisés dans l'invention sont intéressants en raison de leur faible viscosité et de leur aptitude à être durcis par rayonnements X ou électrons, à des doses usuelles (50 à 250 kGy) en donnant un matériau durci ayant une température de transition vitreuse (Tg) élevée, pouvant atteindre 250°C.

Toutefois, le monomère à l'état pur bien qu'il conduise à des propriétés intéressantes, présente l'inconvénient de devenir fragile après durcissement.

Aussi, selon l'invention, on préfère le mélanger à un diluant acrylique monofonctionnel ou aux diacrylates et/ou triacrylates décrits ci-dessus, pour le flexibiliser sans diminuer sa résistance au vieillissement en atmosphère humide.

Les compositions durcissables peuvent comprendre en outre un ou plusieurs autres additifs choisis parmi les agents épaississants, les charges, les colorants, les caoutchoucs et les butadiènes à terminaison vinyle (VTBN). Ces additifs peuvent être utilisés pour régler la viscosité du monomère ou pour améliorer les propriétés de la résine durcie.

Le tétraacrylate à l'état pur présente une viscosité très faible qui peut être intéressante pour réaliser des pièces par moulage par injection, mais qui ne convient pas pour la réalisation de renfort fibreux préimprégné. Dans ce dernier cas, il est nécessaire d'épaissir la formulation par adjonction d'un agent épaississant. Celui-ci peut être une matière thermoplastique ou un agent thixotrope tel que du gel de silice. On peut aussi ajouter des additifs pour améliorer les propriétés mécaniques du matériau, en particulier augmenter sa résistance à la fissuration. Des additifs de ce type peuvent être constitués par un butadiène à terminaison vinyle (VTBN) ou un caoutchouc.

Les compositions durcissables constituées d'un mélange du monomère tétraacrylate avec le diacrylate et/ou triacrylate correspondants, peuvent être obtenues directement lors de la synthèse du tétraacrylate comme on l'a vu ci-dessus, en réglant de façon appropriée les conditions de réaction.

Pour les compositions diacrylate-tétraacrylate, celles-ci peuvent être obtenues par mélange des deux, dans les proportions voulues.

Dans le cas des compositions durcissables à base de tétraacrylate et diacrylate et/ou triacrylate correspondants, la teneur en tétraacrylate doit être suffisante pour que l'on obtienne les propriétés thermiques voulues. Généralement, ces compositions comprennent au moins 50 % en masse de tétraacrylate.

Lorsqu'on utilise un diluant acrylique dans les compositions durcissables de l'invention, celui-ci est choisi en fonction de ses propriétés de flexibilisation de la résine durcie, mais il doit diminuer le Tg dans des proportions acceptables pour les propriétés souhaitées et présenter une bonne miscibilité avec le tétraacrylate. Ce diluant comprend de préférence un ou plusieurs cycles aromatiques ou aliphatiques et une seule fonction acrylique. Parmi les diluants acryliques monofonctionnels de ce type, on peut citer le monoacrylate d'isobornyle.

On peut toutefois utiliser d'autres diluants acryliques, par exemple le diacrylate d'hexanediol et le diacrylate de tripropylène glycol.

Généralement, ce diluant acrylique représente de 30 à 50 % en masse, de préférence de 30 à 40 % en masse de la composition.

Les compositions durcissables peuvent être utilisées pour la préparation de matériaux composites par moulage par injection.

Dans ce cas, le procédé de fabrication du matériau composite consiste à disposer un renfort fibreux dans un moule, à injecter sous pression dans le moule la composition durcissable décrite ci-dessus et à soumettre ensuite le moule rempli de la composition à une ionisation au moyen de rayons X ou d'électrons pour durcir cette composition.

Des doses d'ionisation de 50 à 250 kGy, de préférence de 100 à 150 kGy, conviennent dans ce but. Généralement, on réalise l'ionisation sous atmosphère inerte, par exemple, sous azote.

Bien que les résines acryliques utilisées dans l'invention conviennent plus particulièrement pour la réalisation de matériaux par moulage par injection, elle peuvent être aussi utilisées pour réaliser des matériaux composites par d'autres techniques, par exemple par la technique mettant en oeuvre un renfort fibreux pré- imprégné, sous la forme de fibres revêtues ou de nappes ou tissus préimprégnés que l'on met sous la forme voulue par chauffage, et que l'on durcit ensuite par ionisation au moyen de rayons X ou d'électrons.

Dans ce cas, on utilise une composition comportant un agent épaississant pour préparer le préimprégné.

D'autres caractéristiques et avantages de l'invention apparaitront mieux à la lecture de la description qui suit, d'exemples de réalisation donnés bien entendu à titre illustratif et non limitatif.

### EXEMPLE 1 : Tétraacrylate de formule :

### 1) Préparation.

Lors de la synthèse, deux produits sont formés selon les schémas réactionnels suivants.

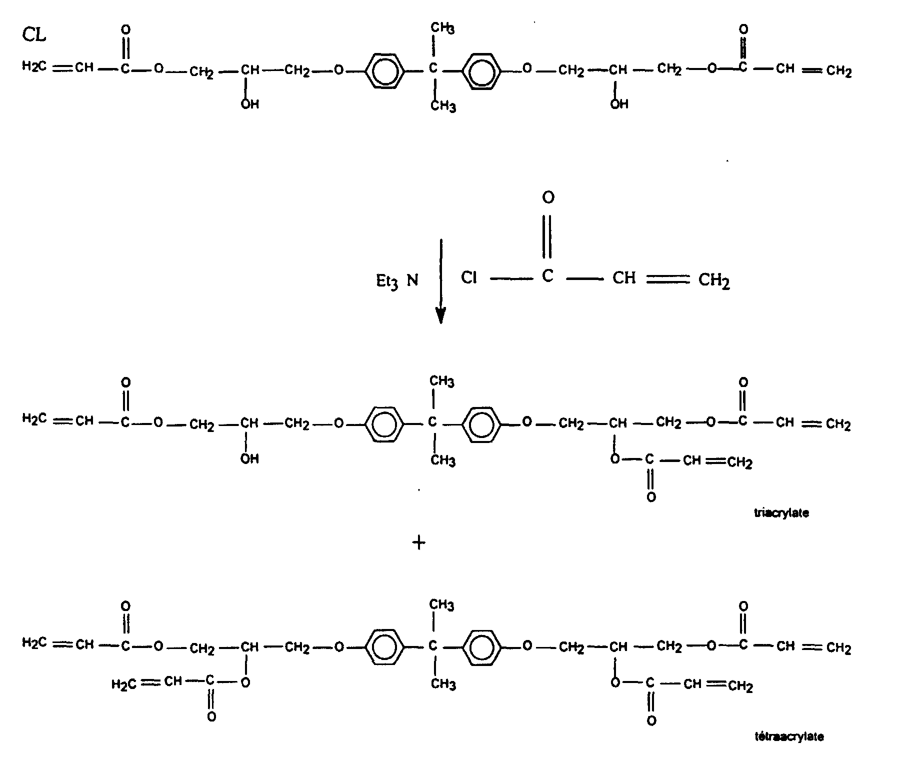

Il est toutefois possible d'obtenir le tétraacrylique pur en modifiant les quantités de réactifs employées (tableau 2)

Dans un tricol de 500 ml, la résine Bis-GA (0,1 mole) dissoute dans 300 ml de chloroforme anhydre est mélangée avec de la triéthylamine (0,4 mole) dans 150 ml de chloroforme anhydre. Le montage est placé sous atmosphère inerte (balayage d'azote). Le chlorure d'acryloyle (0,37 mole) dans 150 ml de chloroforme anhydre, est ajouté (à température ambiante) rapidement afin d'obtenir le composé tétraacrylique pur. Lorsque l'on opère lentement, on obtient un mélange de triacrylate et tétraacrylate et la vitesse d'addition est déterminante sur les proportions des produits formés. Le mélange est agité pendant 20 heures. La solution se colore en jaune. Le sel de triethylamine formé au cours de la réaction précipite dans le milieu lorsque de l'éther est versé sur la solution. Après filtration et évaporation des solvants, la résine obtenue est le tétraacrylate.

### 2) Durcissement

On dispose par exemple dans des moules ouverts paralléllépidiques (100/40/2 mm) le tétraacrylate obtenu précédemment, puis on dégaze sous vide et on soumet les échantillons ainsi préparés à l'action d'un faisceau d'électrons engendré par un accélérateur.

Les conditions d'ionisation sont les suivantes :
- puissance : 20 kW
- Energie moyenne du faisceau : 10 MeV
- atmosphère : azote
- dose : 150 à 250 kGy.

On obtient ainsi des échantillons durcis, et on détermine les températures de transition vitreuse (Tg) de ceux-ci. Les résultats obtenus en fonction de la dose sont donnés dans le tableau 1 qui suit.

**TABLEAU 1**

| **Monomère** | **Dose (kGy)** | **Tg (°C)** |
|---|---|---|
| Tétraacrylate pur | 150 | 210 |
| | 200 | 230 |
| | 250 | 248 |

Les Tg obtenus sont élevés, quelle que soit la dose utilisée. Par ailleurs, dans tous les cas, l'action d'un post-traitement thermique n'apporte pas d'amélioration des propriétés des matériaux ; ceci indique qu'ils sont convenablement polymérisés.

Les caractérisques mécaniques des échantillons durcis sont les suivantes :
- module : 3700 MPa
- contrainte à la rupture : 45 MPa
- pourcentage d'allongement : 1,2 %.

Ces matériaux formés à partir de tétraacrylate à l'état pur ont ainsi de bonnes propriétés. Toutefois, en raison de leur grande rigidité, il risque de se produire des fractures lors du vieillissement en atmosphère humide. Il est donc préférable de diluer le tétraacrylate avec un autre monomère acrylique pour le flexibiliser.

### EXEMPLES 2 à 5 : Compositions durcissables à base de mélanges de tétraacrylate et de di ou triacrylate.

Les compositions des exemples 2 à 5 sont données dans le tableau 2. Les compositions des exemples 4 et 5 sont obtenues en mélangeant le tétraacrylate préparé dans l'exemple 1 avec le diacrylate correspondant dans les proportions indiquées dans le tableau 2.

Les compositions des exemples 2 et 3 sont obtenues directement par synthèse en opérant comme dans l'exemple 1, mais en utilisant les quantités de réactifs données dans le tableau 3 et en ajoutant lentement le chlorure d'acryloyle.

On soumet les compositions à un durcissement comme dans l'exemple 1, en utilisant une dose d'ionisation de 100 ou de 150 kGy.

Les caractéristiques des produits durcis obtenus sont données dans le tableau 2.

**TABLEAU 2**

| **Ex** | **Composition (% en poids)** | **Dose (kGy)** | **Tg °C** | **Module (MPa)** | **Allongement %** |
|---|---|---|---|---|---|
| 1 | Tétraacrylate pur | 150 | 210 | 3700 | 1,2 |
| 2 | Tétraacrylate¹⁾ - triacrylate ²⁾ | | | | |
| | 90 % 10 % | 150 | 160 | 3300 | 1,6 |
| 3 | Tétraacrylate¹⁾- triacrylate ²⁾ | | | | |
| | 80 % 20 % | 150 | 150 | 3700 | 1,8 |
| 4 | Tétraacrylate¹⁾-diacrylate ³⁾ | | | | |
| | 50 % 50 % | 100 | 173 | / | / |
| 5 | Tétraacrylate¹⁾-diacrylate ³⁾ | | | | |
| | 25 % 75 % | 100 | 170 | / | / |

| | | | | | |
|---|---|---|---|---|---|
| 1) tétraacrylate de l'exemple 1 | | | | | |
| 2) triacrylate ayant la formule donnée dans l'exemple 1. | | | | | |
| 3) diacrylate ou bis-GA ayant la formule donnée dans l'exemple 1. | | | | | |

**TABLEAU 3**

| Ex | Formulation | Réactifs | Nombre de mole |
|---|---|---|---|
| 1 | Tétraacrylique pur | Bis-GA | 0,1 |
| | | Chlorure d'acryloyle | 0,37 |
| | | Triéthylamine | 0,4 |
| 2 | Tétra(90) / Triacrylate (10) | Bis-GA | 0,1 |
| | | Chlorure d'acryloyle | 0,32 |
| | | Triéthylamine | 0,34 |
| 3 | Tétra (80) / Triacrylate (20) | Bis-GA | 0,1 |
| | | Chlorure d'acryloyle | 0,28 |
| | | Triéthylamine | 0,3 |

Au vu du tableau 2, on remarque que l'ajout de diacrylate ou triacrylate apporte de la flexibilisation, mais diminue la température de transition vitreuse.

### EXEMPLE 6 à 9 : Compositions durcissables à base de tétraacrylate et d'un diluant monoacrylique.

Dans ces exemples, on utilise le tétraacrylate de l'exemple 1 et un diluant réactif constitué par du monoacrylate d'isobornyle en proportion telle qu'il représente 10, 20, 30 ou 40 % en masse de la composition.

A partir de ces compositions, on réalise des produits durcis en suivant le même mode opératoire que dans l'exemple 1, et en utilisant une dose de 150 kGy.

La température de transition vitreuse (Tg) des produits obtenus après durcissement et les caractéristiques (Tg et % d'eau absorbée) obtenues après vieillissement du produit durci en atmosphère humide, sont données dans le tableau 4 qui suit.

Le vieillissement en atmosphère humide est effectué selon la norme avionique SACMA SRM 11-88 ; il consiste en une immersion des échantillons de matériaux durcis dans l'eau distillée à 70°C pendant 336 heures.

**TABLEAU 4**

| **Ex** | **Composition durcissable** | **Teneur en AIB* (% en poids)** | **Dose (kGy)** | **Tg (°C)** | **Tg après vieillisseement (°C)** | **% eau absorbée** |
|---|---|---|---|---|---|---|
| 6 | tétraacrylate + Acrylate d'isobornyle | 10 | 150 | 65 et 190 | Matériau dégradé | Matériau dégradé |
| 7 | tétraacrylate + Acrylate d'isobornyle | 20 | 150 | 200 | Matériau dégradé | Matériau dégradé |
| 8 | tétraacrylate + Acrylate d'isobornyle | 30 | 150 | 195 | 180 | 3,4 |
| 9 | tétraacrylate + Acrylate d'isobornyle | 40 | 150 | 205 | 190 | 2,5 |

| | | | | | | |
|---|---|---|---|---|---|---|
| *AIB = Acrylate d'isobornyle | | | | | | |

Les résultats de ce tableau montrent qu'il convient d'utiliser au moins 30 % de diluant monoacrylique pour obtenir un matériau sain après vieillissement humide. Les températures de transition vitreuse des matériaux durcis sont d'environ 200°C et les pourcentages d'eau absorbée au cours du vieillissement en atmosphère humide qui sont de l'ordre de 2 %, sont plus faibles que ceux des résines époxyacryliques qui, en général, absorbent autour de 5 % d'eau.

### EXEMPLES 10 à 12 : Compositions durcissables avec diluants monoacryliques.

Dans ces exemples, on suit le même mode opératoire que dans les exemples 6 à 9 pour préparer des matériaux durcis à partir de compositions durcissables comprenant 30 % en masse, 35 % en masse ou 40 % en masse d'acrylate d'isobornyle (AIB) et des doses données dans le tableau 5 qui suit.

Dans ce tableau 5, on a également reporté les propriétés mécaniques des produits durcis.

**TABLEAU 5**

| Ex | Formulation ( % en poids) | Dose (kGy) | Module (MPa) | Contrainte maximale (MPa) | Allongement ( %) |
|---|---|---|---|---|---|
| 10 | Tétra + AIB* | | | | |
| | 70 - 30 | 150 | 3500 | 42 | 1,2 |
| 11 | Tétra + AIB* | | | | |
| | 65 - 35 | 150 | 3700 | 72 | 2 |
| 12 | Tétra + AIB* | | | | |
| | 60 - 40 | 150 | 3500 | 63 | 1,7 |

| | | | | | |
|---|---|---|---|---|---|
| *AIB = Acrylate d'isobornyle | | | | | |

On remarque ainsi que ces compositions permettent l'élaboration d'une matrice de matériau composite ayant des caractéristiques satisfaisantes.

### EXEMPLES 13 à 18 : Matériaux composites.

Dans ces exemples, on prépare des matériaux composites à partir d'un renfort fibreux constitué de tissus de carbone que l'on dispose dans un moule, imprégnés d'une composition durcissable ayant la formulation donnée dans le tableau 6 qui suit, puis on durcit sous vide par ionisation au moyen d'électrons en utilisant la dose indiquée également dans le tableau 6.

Les températures de transition vitreuse (Tg) avant et après vieillissement du matériau en atmosphère humide, conformément à la norme avionique SACMA/SRM11-88, sont données dans le tableau 6.

**TABLEAU 6**

| **EX** | **Composition durcissable** | **Dose (kGy)** | **Tg (°C)** | **Tg après vieillissement (°C)** |
|---|---|---|---|---|
| 13 | Tétraacrylate + 30 % AIB* | 100 | 240 | 252 |
| 14 | Tétraacrylate + 30 % AIB | 150 | 236 | 253 |
| 15 | Tétraacrylate + 35 % AIB | 100 | 245 | 244 |
| 16 | Tétraacrylate + 35 % AIB | 150 | 250 | 250 |
| 17 | Tétraacrylate + 40 % AIB | 100 | 238 | 246 |
| 18 | Tétraacrylate + 40 % AIB | 150 | 242 | 244 |

| | | | | |
|---|---|---|---|---|
| *AIB = Acrylate d'isobornyle | | | | |

Les résultats du tableau 6 confirment l'obtention de matériaux présentant des températures de transition vitreuse supérieures à 200°C, susceptibles d'avoir un comportement en atmosphère humide répondant aux exigences aéronautiques.

Par ailleurs, si l'on compare ces résultats avec ceux des tableaux 3 et 4, on remarque que les Tg des matériaux composites sont supérieurs à ceux des compositions de résine seule durcie. Ceci confirme l'intérêt des compositions qui sont suffisamment réactives pour apporter leurs propriétés à un matériau dans lequel elles ne représentent que 30 % en volume.

## Revendications

1. Matériau composite comprenant un renfort fibreux et une matrice obtenue par polymérisation d'une composition durcissable par ionisation au moyen de rayons X ou d'électrons, constituée par un mélange d'un tétraacrylate de formule : 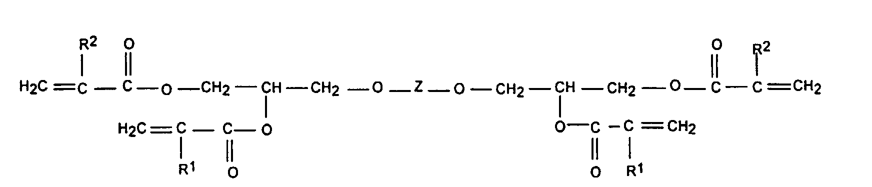 dans laquelle R¹ et R² qui peuvent être identiques ou différents, représentent H ou CH₃, et Z est un groupement bivalent comportant un ou deux cycles aromatiques ou aliphatiques,
avec
1) un diacrylate et/ou triacrylate de formule : 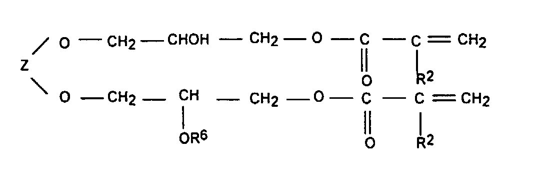 dans laquelle Z et R² ont les significations données ci-dessus et R⁶ représente H ou le groupe de formule : 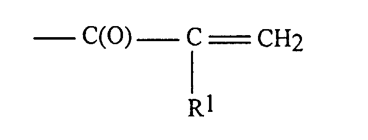 dans laquelle R¹ représente H ou CH₃, ou
2) un diluant acrylique monofonctionnel.

2. Matériau selon la revendication 1, caractérisé en ce que le renfort est formé de fibres de carbone, de fibres de verres ou de fibres d'aramide.

3. Matériau composite selon l'une quelconque des revendications 1 et 2, caractérisé en ce que le renfort fibreux représente de 55 à 75% en volume du matériau.

4. Matériau composite selon l'une quelconque des revendications 1 à 3, caractérisé en ce que, dans la formule du tétraacrylate Z représente un groupe de formule :
-R³-R⁴-R³-
dans laquelle R⁴ représente : 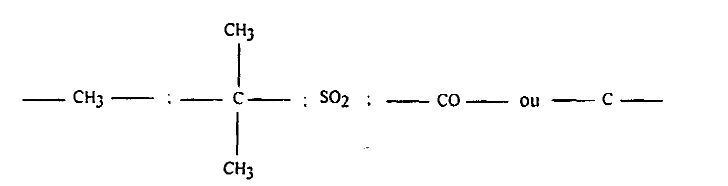 et les R³ sont des cycles aromatiques.

5. Matériau composite selon la revendication 4, caractérisé en ce que Z représente un groupe de formule : 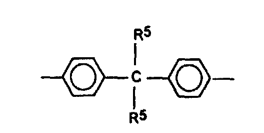 dans laquelle R⁵ représente un atome d'hydrogène ou un groupe méthyle.

6. Matériau selon l'une quelconque des revendications 1 à 3, caractérisé en ce que le tétraacrylate répond à la formule :

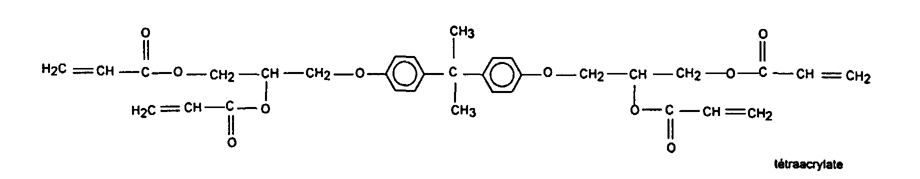

7. Matériau selon l'une quelconque des revendications 1 à 6, caractérisé en ce que le diluant acrylique est le monoacrylate d'isobornyle.

8. Procédé de préparation d'un matériau composite caractérisé en ce qu'il consiste à disposer un renfort fibreux dans un moule, à injecter sous pression dans le moule une composition durcissable constituée par un mélange d'un tétraacrylate de formule : 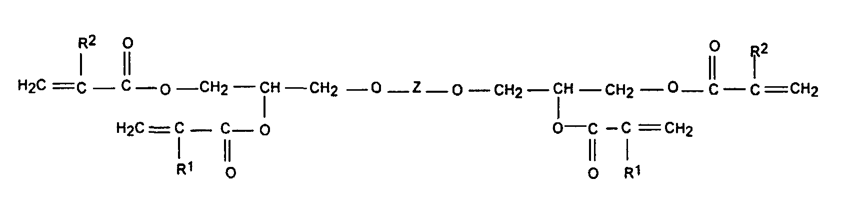 dans laquelle R¹ et R² qui peuvent être identiques ou différents, représentent H ou CH₃, et Z est un groupement bivalent comportant un ou deux cycles aromatiques ou aliphatiques,
avec
1) un diacrylate et/ou triacrylate de formule : 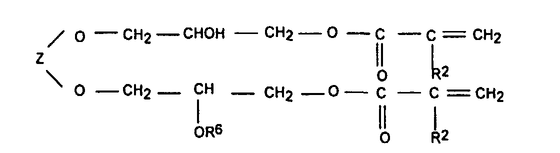 dans laquelle Z et R² ont les significations données ci-dessus et R⁶ représente H ou le groupe de formule : 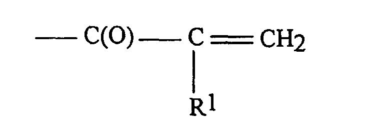 dans laquelle R¹ représente H ou CH₃, ou
2) un diluant acrylique monofonctionnel, et à soumettre ensuite le moule rempli de la composition à une ionisation au moyen de rayons X ou d'électrons pour durcir la composition.

9. Procédé selon la revendication 8, caractérisé en ce que la composition durcissable est un mélange de tétraacrylate et de diluant acrylique monofonctionnel et en ce qu'elle comprend de 50 à 70% en masse de tétraacrylate.

10. Procédé selon la revendication 8 ou 9, caractérisé en ce que le diluant monofonctionnel est le monoacrylate d'isobornyle.

11. Procédé selon la revendication 8, caractérisé en ce que la composition est un mélange de tétraacrylate et de diacrylate et/ou triacrylate et en ce qu'elle comprend au moins 50% en masse de trétraacrylate.

12. Procédé selon l'une quelconque des revendications 8 à 11, caractérisé en ce que la composition durcissable comprend en outre un ou plusieurs additifs choisis parmi les agents épaississants, les charges, les colorants, les caoutchoucs et les butadiènes à terminaisons vinyle.

13. Procédé selon l'une quelconque des revendications 8 à 12, caractérisé en ce que, dans la formule du tétraacrylate, Z représente un groupe de formule :
-R³-R⁴-R³-
dans laquelle R⁴ représente : 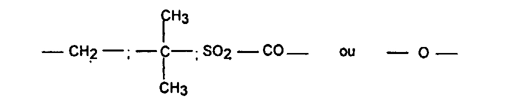 et les R³ sont des cycles aromatiques.

14. Procédé selon la revendication 13, caractérisé en ce que Z représente un groupe de formule : 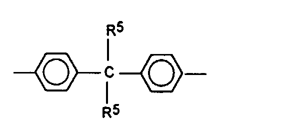 dans laquelle R⁵ représente un atome d'hydrogène ou un groupe méthyle.

15. Procédé selon la revendication 14, caractérisé en ce que le tétraacrylate répond à la formule :

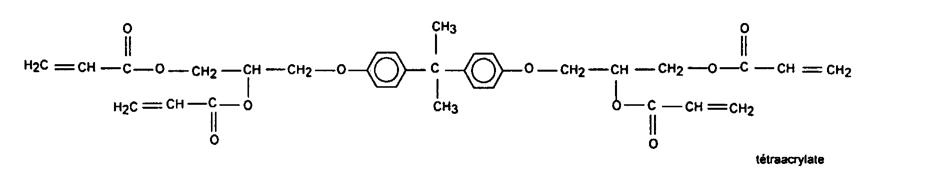

## Patentansprüche

1. Verbundmaterial, enthaltend eine faserige Verstärkung und eine Matrix, welche gewonnen ist durch Polymerisation einer durch Ionisation mit Hilfe von Röntgen- oder Elektronenstrahlen härtbaren Zusammensetzung, die aus einer Mischung eines Tetraacrylats der Formel: 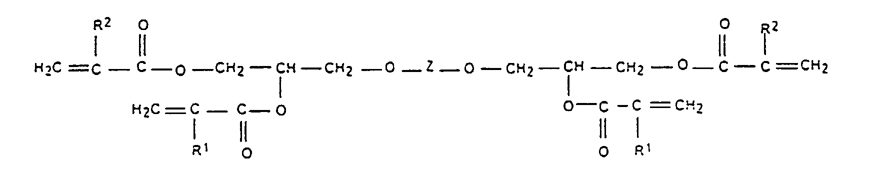 worin R¹ und R², die gleich oder verschieden sein können, H oder CH₃ darstellen und Z eine zweiwertige Gruppierung ist, die einen oder zwei aromatische oder aliphatische Ringe enthält, mit
1) einem Diacrylat und/oder Triacrylat der Formel: 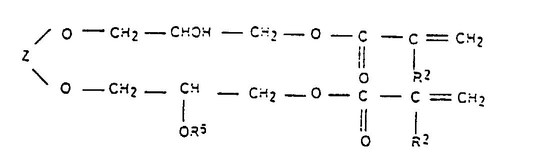 worin Z und R² die oben gegebenen Bedeutungen haben und R⁶ für H oder die Gruppe der Formel: 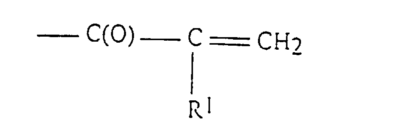 steht, worin R¹ H oder CH₃ bedeutet, oder mit
2) einem monofunktionellen Acryl-Verdünnungsmittel besteht.

2. Material nach Anspruch 1, dadurch gekennzeichnet, daß die Verstärkung aus Kohlenstoffasern, Glasfasern oder Aramidfasern gebildet ist.

3. Verbundmaterial nach einem der Ansprüche 1 und 2, dadurch gekennzeichnet, daß die faserige Verstärkung 55 bis 75 Vol.-% des Materials darstellt.

4. Verbundmaterial nach einem der Ansprüche 1 bis 3, dadurch gekennzeichnet, daß in der Formel des Tetraacrylats Z eine Gruppe der Formel:
-R³-R⁴-R³-
darstellt, worin R⁴ 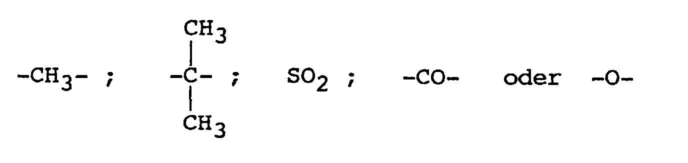 bedeutet und die R³ aromatische Ringe sind.

5. Verbundmaterial nach Anspruch 4, dadurch gekennzeichnet, daß Z eine Gruppe der Formel: 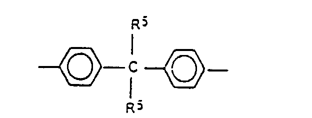 darstellt, worin R⁵ für ein Wasserstoffatom oder eine Methylgruppe steht.

6. Material nach einem der Ansprüche 1 bis 3, dadurch gekennzeichnet, daß das Tetraacrylat der Formel: 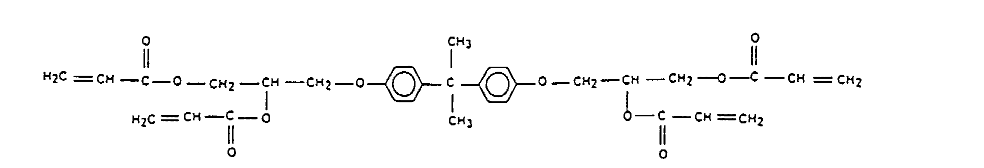 entspricht.

7. Material nach einem der Ansprüche 1 bis 6, dadurch gekennzeichnet, daß das Acryl-Verdünnungsmittel Isobornyl-monoacrylat ist.

8. Verfahren zur Herstellung eines Verbundmaterials, dadurch gekennzeichnet, daß es darin besteht, eine faserige Verstärkung in eine Form zu legen, in die Form unter Druck eine härtbare Zusammensetzung zu injizieren, die aus einer Mischung eines Tetraacrylats der Formel: 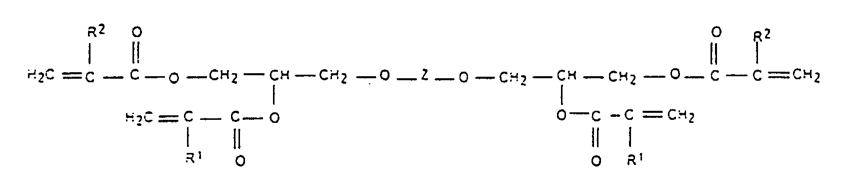 worin R¹ und R², die gleich oder verschieden sein können, H oder CH₃ darstellen und Z eine zweiwertige Gruppierung ist, die einen oder zwei aromatische oder aliphatische Ringe enthält,
mit 1) einem Diacrylat und/oder Triacrylat der Formel: 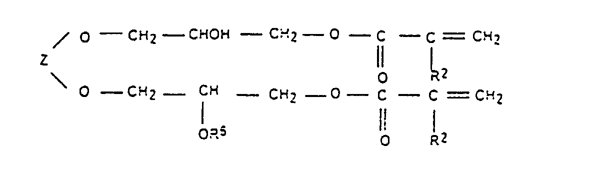 worin Z und R² die oben gegebenen Bedeutungen haben und R⁶ für H oder die Gruppe der Formel: 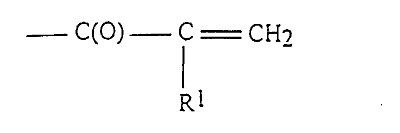 steht, worin R¹ H oder CH₃ bedeutet, oder mit
2) einem monofunktionellen Acryl-Verdünnungsmittel besteht, und anschließend die mit der Zusammensetzung gefüllte Form mit Hilfe von Röntgen- oder Elektronenstrahlen einer Ionisierung zu unterziehen, um die Zusammensetzung zu härten.

9. Verfahren nach Anspruch 8, dadurch gekennzeichnet, daß die härtbare Zusammensetzung eine Mischung von Tetraacrylat und monofunktionellem Acryl-Verdünnungsmittel ist und daß sie 50 bis 70 Massen-% Tetraacrylat enthält.

10. Verfahren nach Anspruch 8 oder 9, dadurch gekennzeichnet, daß das monofunktionelle Verdünnungsmittel Isobornyl-monoacrylat ist.

11. Verfahren nach Anspruch 8, dadurch gekennzeichnet, daß die Zusammensetzung eine Mischung von Tetraacrylat und von Diacrylat und/oder Triacrylat ist und daß sie mindestens 50 Massen-% Tetraacrylat enthält.

12. Verfahren nach einem der Ansprüche 8 bis 11, dadurch gekennzeichnet, daß die härtbare Zusammensetzung ferner ein oder mehrere Additive enthält, die unter den Verdickungsmitteln, den Füllstoffen, den Farbstoffen, den Kautschuken und den Butadienen mit Vinyl-Endgruppen gewählt sind.

13. Verfahren nach einem der Ansprüche 8 bis 12, dadurch gekennzeichnet, daß in der Formel des Tetraacrylats Z eine Gruppe der Formel:
-R³-R⁴-R³-
darstellt, worin R⁴ 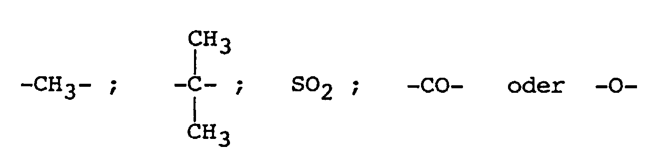 bedeutet und die R³ aromatische Ringe sind.

14. Verfahren nach Anspruch 13, dadurch gekennzeichnet, daß Z eine Gruppe der Formel: 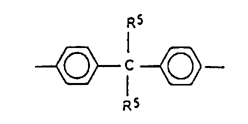 darstellt, worin R⁵ für ein Wasserstoffatom oder eine Methylgruppe steht.

15. Verfahren nach Anspruch 14, dadurch gekennzeichnet, daß das Tetraacrylat der Formel: 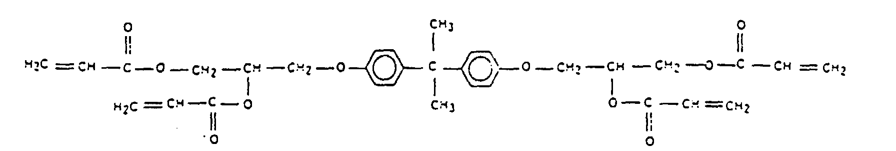 entspricht.

## Claims

1. Composite material comprising a fibrous reinforcement and a matrix obtained by the polymerization of a composition hardenable by ionization by means of X-rays or electrons, constituted by a mixture of a tetraacrylate of formula: 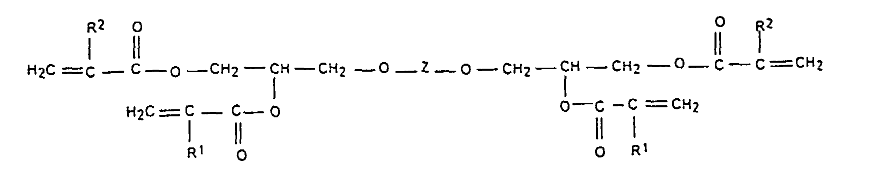 in which R¹ and R², which can be the same or different, represent H or CH₃ and Z is a divalent group having one or two aromatic or aliphatic cycles, with
1) a diacrylate and/or triacrylate of formula: 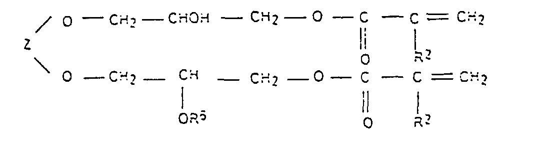 in which Z and R² have the meanings given hereinbefore and R⁶ represents H or the group of formula: 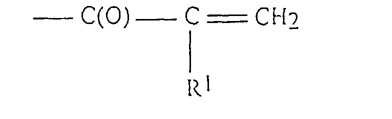 in which R¹ represents H or CH₃, or
2) a monofunctional acrylic diluent.

2. Material according to claim 1, characterized in that the reinforcement is formed from carbon fibres, glass fibres or aramide fibres.

3. Composite material according to either of the claims 1 and 2, characterized in that the fibrous reinforcement represents 55 to 75 vol.% of the material.

4. Composite material according to any one of the claims 1 to 3, characterized in that, in the formula of the tetraacrylate, Z represents a group of formula:
-R³-R⁴-R³-
in which R⁴ represents: 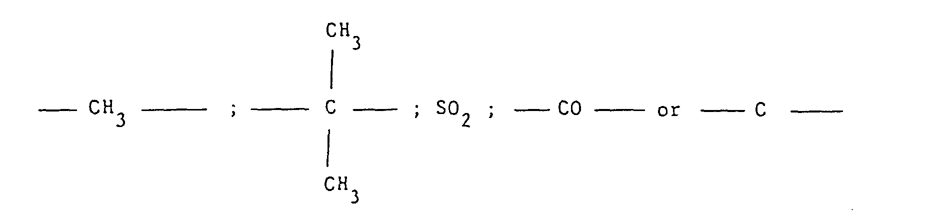 and the R³ are aromatic cycles.

5. Composite material according to claim 4, characterized in that Z represents a group of formula: 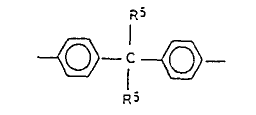 in which R⁵ represents a hydrogen atom or a methyl group.

6. Material according to any one of the claims 1 to 3, characterized in that the tetraacrylate complies with the formula:

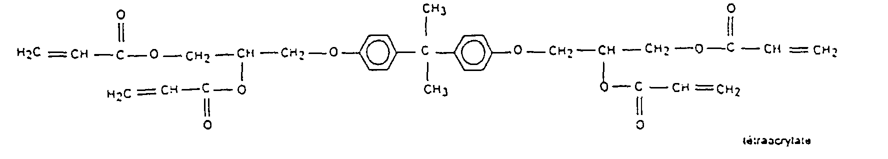

7. Material according to any one of the claims 1 to 6, characterized in that the acrylic diluent is isobornyl monoacrylate.

8. Process for the preparation of a composite material, characterized in that it consists of placing a fibrous reinforcement in a mould, injecting under pressure into said mould a hardenable composition constituted by a mixture of a tetracrylate of formula: 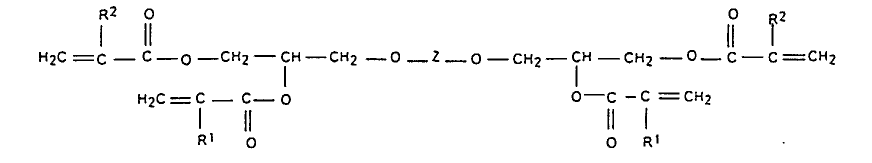 in which R¹ and R², which can be the same or different, represent H or CH₃ and Z is a divalent group having 1 or 2 aromatic or aliphatic cycles, with
1) a diacrylate and/or triacrylate of formula: 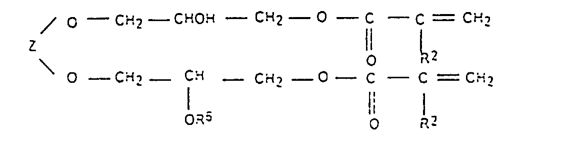 in which Z and R² have the meanings given hereinbefore and R⁶ represents H or the group of formula: 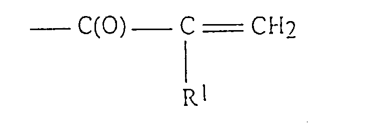 in which R¹ represents H or CH₃, or
2) a monofunctional acrylic diluent, and then subjecting the mould filled with the composition to an ionization by means of X-rays or electrons in order to harden the composition.

9. Process according to claim 8, characterized in that the hardenable composition is a mixture of tetraacrylate and a monofunctional acrylic diluent and in that it comprises 50 to 70 wt.% tetraacrylate.

10. Process according to claim 8 or 9, characterized in that the monofunctional diluent is isobornyl monoacrylate.

11. Process according to claim 8, characterized in that the composition is a mixture of tetraacrylate and diacrylate and/or triacrylate and in that it comprises at least 50 wt.% tetraacrylate.

12. Process according to any one of the claims 8 to 11, characterized in that the hardenable composition also comprises one or more additives chosen from among thickening agents, fillers, dyes, rubbers and vinyl termination butadienes.

13. Process according to any one of the claims 8 to 12, characterized in that, in the formula of the tetraacrylate, Z represents a group of formula:
-R³-R⁴-R³-
in which R⁴ represents: 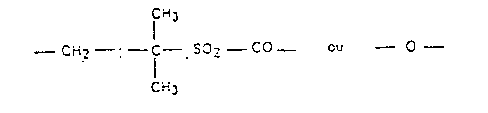 and the R³ are aromatic cycles.

14. Process according to claim 13, characterized in that Z represents a group of formula: 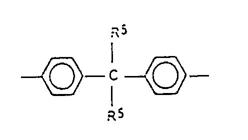 in which R⁵ represents a hydrogen atom or a methyl group.

15. Process according to claim 14, characterized in that the tetraacrylate is in accordance with the formula:
